# EUROPEAN PATENT SPECIFICATION

(11) **EP 2 856 954 B1**
(45) Date of publication and mention of the grant of the patent: **21.06.2017**
(21) Application number: 12877095.5
(22) Date of filing: 26.10.2012
(51) Int. Cl.: A61B 17/326, A61B 17/115

(54) **DISPOSABLE CIRCUMCISING ANASTOMAT**
EINWEG-BESCHNEIDUNGSANASTOMAT
DISPOSITIF D'ANASTOMOSE JETABLE POUR LA CIRCONCISION

(30) Priority: 28.05.2012 CN 201210169127
(43) Date of publication of application: 08.04.2015
(73) Proprietor: Shang, Jianzhong, Wuhu, Anhui 241000 (CN)
(72) Inventor: Shang, Jianzhong, Wuhu, Anhui 241000 (CN)
(74) Representative: Harris, Oliver John Richard
(86) International application number: PCT/CN2012/001438
(87) International publication number: WO 2013/173954

(56) References cited:
- EP-A1- 2 305 146
- CN-A- 101 983 615
- CN-A- 101 991 456
- CN-A- 102 068 297
- CN-A- 102 068 297
- CN-A- 102 309 357
- CN-A- 102 309 358
- CN-A- 102 309 358
- CN-A- 102 327 140
- CN-A- 102 451 031
- CN-A- 102 451 031
- CN-A- 102 743 211
- CN-U- 202 604 965
- CN-Y- 2 694 913
- US-A- 5 797 921

## Description

### Field of the Invention

The invention relates to a type of apparatus for fitly circumcising a penis, especially a type of circumcision apparatus that prevents a fixture device from opening during a circumcision procedure.

### Background

The effect of male circumcision has been widely recognized in reducing the transmission of HIV. Mr. Jianzhong Shang has previously invented a disposable circumcision apparatus that utilizes upper and lower blades that join to form a circular clamp, the blades staggered from one another, so as to minimize pain resulting from skin clamping during the procedure. This technique has been improved over the years to further ensure the convenience, safety, and effectiveness of this operation on a wide range of patients. This circumcision apparatus is the present standard in circumcision surgery.

The disposable circumcision device mentioned above comprises a fixture device (exterior ring) and balanus ferrule (interior ring), which are made of hard, non-toxic medical plastics. A patient may experience pain and discomfort during surgery as a result of the hard, circular clamp and balanus ferrule directly contacting the prepuce.

One problem with the prior art circumcision device mentioned above is the possibility of dropping the device during the procedure, which may directly lead to bleeding. Additionally, a patient may be fearful of such an accidental during the circumcision procedure and cause unnecessary mental anguish prior to the procedure. Some have attempted to reduce the chances of dropping a circumcision apparatus during the procedure by using scalariform detent blocks at each end of the circumcision apparatus, so as to prevent the accidental opening of the fixture device that may lead to dropping the entire apparatus. The current Shanghuan Brand circumcision apparatus uses this technique.

It would be desirable to avoid the pain and discomfort associated with the prepuce being in direct contact with the circular blades of the fixture device and balanus ferrule. CN 102451031 A1 discloses a disposable circumcision anastomat in accordance with the preamble of claim 1.

### SUMMARY

The main purpose of this invention is to overcome the deficiencies of existing circumcision devices and to provide a safer, cleaner, and more convenient disposable circumcision apparatus. Claim 1 defines the invention and the dependent claims disclose the preferred embodiments.

In accordance with the present invention there is provided a disposable circumcision apparatus as defined in claim 1.

The first embodiment is that, the blade further comprises a groove formed along the blade interior circumferential edge for receiving the cushion ring.

The second embodiment is that, the cushion ring comprises a groove formed along a circumference of the cushion ring for receiving the blade interior circumferential edge or the entire blade is placed within the groove. Wherein the cushion ring is made of silica-gel and the blade is made of plastic, and the cushion ring adheres to the blade through an integral formation between plastics and silica-gel. The fixture device is coated with a silica-gel layer, and the silica-gel layer adheres to the fixture device through an integral formation between plastics and silica-gel.

Where in, the blade interior circumferential edge thickness is greater than or equal to the thickness of exterior circumferential edge of the blade where the blade contacts an interior circumferential surface of the fixture device. A circular elastic pad, wherein the elastic pad is placed around the exterior of the balanus ferrule. A first rim located on a first side of the balanus ferrule; and a second rim locatged on a second side of the balanus ferrule; wherein the first and second rims forma ring-shaped cavity upon which the elastic pad rests. The cushion ring and the elastic pad comprises a micro mechanism for preventing skin adhesion to the cushion ring and the elastic pad. The micro mechanism is selected from the group consisting of sinking and bulging dots, crossed sinking and bulging lines, grooves, protrusions, and an uneven structure. The fixture device and the balanus ferrule comprise an elliptic or obliquely elliptic structure corresponding to a cross section of a coronal sulcus. The balanus ferrule further comprises a notch for accommodating a frenulum of prepuce.The fixture device is C-shaped. The fixture device comprises three sections connected together at two hinge points, the hinge points staggered from each other so that they minimize contact with a scrotum during circumcision surgery. The means for maintaining the fixture device in a closed position comprises upper and lower scalariform detent blocks, each of the scalariform detent blocks comprising a hook and a hole, wherein the hooks are sized and shaped to fit within the holes, respectively. The buckling device comprises a screw on one end of the fixture device and a linear threaded hole on the other end of the fixture device. Comprising a protective cover sized and shaped to fit over the buckling device. The cross-section of the groove is selected from the group consisting of an arc, a semi-circle, a semi-ellipse, and a triangle; and the cross-section of the first blade mating portion and the second blade mating portion combined is selected from the group consisting of an arc, a semi-circle, a semi-ellipse, and a triangle; and the cushion ring comprising a cross section substantially matching the cross section of the groove. The cushion ring is of ring shape and the circumference thereof corresponds with that of the blade. The fixture device comprises one, two, or three blades. Round angles are provided at the first and second blade mating portions.

The beneficial effects of the invention are as follows:
1. An interior circumferential edge of each blade has a groove for retaining a cushion ring, such as a non-toxic medical silica-gel cushion ring, which clamps directly on the prepuce. A soft elastic pad is also provided on the surface of a balanus ferrule. Therefore, both the outer and inner surfaces of prepuce touch a soft material, providing some space for expansion and effectively alleviating the pain and discomfort caused by prepuce irritation against the prior art hard surfaces of the half-ring and balanus ferrule.
2. A groove is provided on the inner side of the blade, increasing the softness of the surface while still keeping the circumcision device in place.
3. The first blade mating portion and the second blade mating portion are procided at the open end of the blade, respectively. These portions of the blade are designed to slide over each other, or overlap, to form a complete blade structure (e.g., ring) as the fixture device is placed into a closed position. The first and second blade better facilitate clamping of the fixture device around the foreskin of the penis during surgery. When in use, the cushion ring will neither be obstructed at the fixture device opening due to wrinkling nor clamp the prepuce during the procedure when the upper and second blade come in contact with each other.
4. A micro mechanism preventing skin adhesion may be provided for both the cushion ring and the elastic pad, so as to reduce the possibility of skin adhesion of the prepuce, reducing pain associated with the circumcision apparatus removal process.
5. A hook and a linear groove may be provided on each side of a buckling device located on each open end of the fixture device, and a protective cover may be provided over the buckling device, minimizing the risk of the buckling device opening due to, for example, a penile erection.
6. The cross section of both the fixture device and balanus ferrule may form an elliptic or obliquely elliptic structure, which may more closely resemble the cross section of an actual penis, especially the shape of the coronal sulcus. The pain resulting from irritating the frenulum during an erection is further reduced.
7. The fixture device may comprise a one-section, two-section, or three-section ring-shaped apparatus. The one-section fixture device may be made exclusively of soft materials, has an adjustable opening and is simple and convenient. The two-section fixture is typically made of hard, non-toxic medical plastics that are rotatably joined together at one end, forming an adjustable opening at the other. In this embodiment, the fixture device is rigid, ensuring the structural integrity of the circumcision apparatus. Additionally, this embodiment allows for a large opening formed at the "open" ends of the two semi-ferrules via the hinge structure formed by at the two, opposing ends of the semi-ferrules. During surgery, the connecting ends are typically located above of the scrotum, causing friction between the hinge and the scrotum, thereby causing patient discomfort. The three-section design may alleviate this problem, because two pivot points are staggered from each other so that contact with the scrotum is reduced or completely eliminated.
8. A notch for receiving the frenulum may be provided within the balanus ferrule, shaped to correspond with the penis structure and preventing pressure on the frenulum.

### Brief Description of the Drawings

Fig 1A is an exploded view of an apparatus for circumcising a penis, comprising a fixture device, balanus ferrule, cushion ring, and circular elastic pad;
Fig 1B illustrates the fixture device and cushion ring of Fig 1A interconnected with one another;
Fig 1C is a perspective view of the apparatus of Fig 1A in a closed position;
Fig 1D illustrates cross-section A-A shown in Fig 1C;
Figs 2A and 2B illustrate left and right semi-ferrules, respectively, of the fixture device shown in Fig 1A;
Figs 2C, 2D, and 2E illustrate vertical cross-sections of three different embodiments of the fixture device shown in Fig 1A, respectively;
Figs 2F, 2G, and 2H illustrate first and second mating half-ring portions of three different embodiments of the fixture device shown in Figs 2C, 2D, and 2E, respectively;
Figs 21 and 2J illustrate a cross-section of two embodiments of the fixture device of Fig 1A, each having two blade;

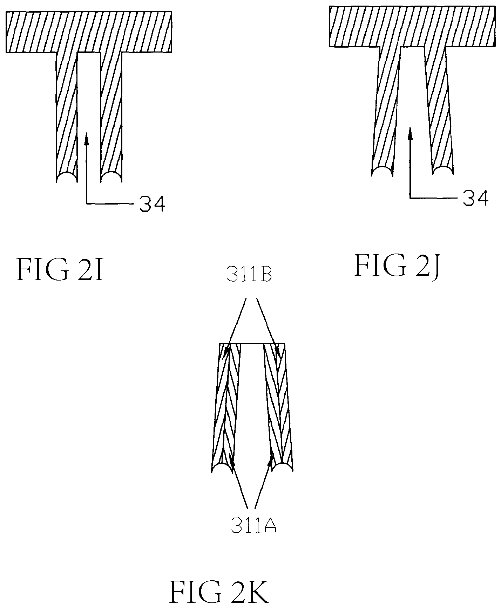

Fig 2K is an illustration of first and second mating blade portions for the structure of Fig 2J;

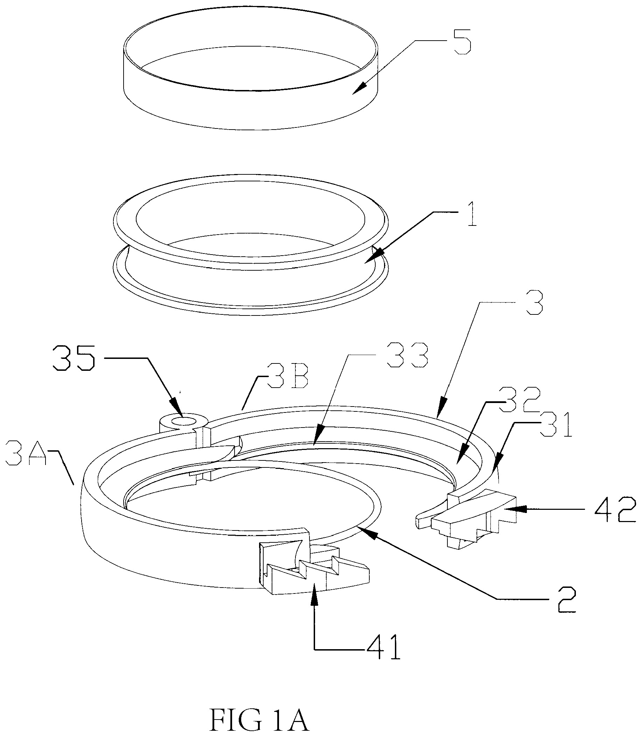

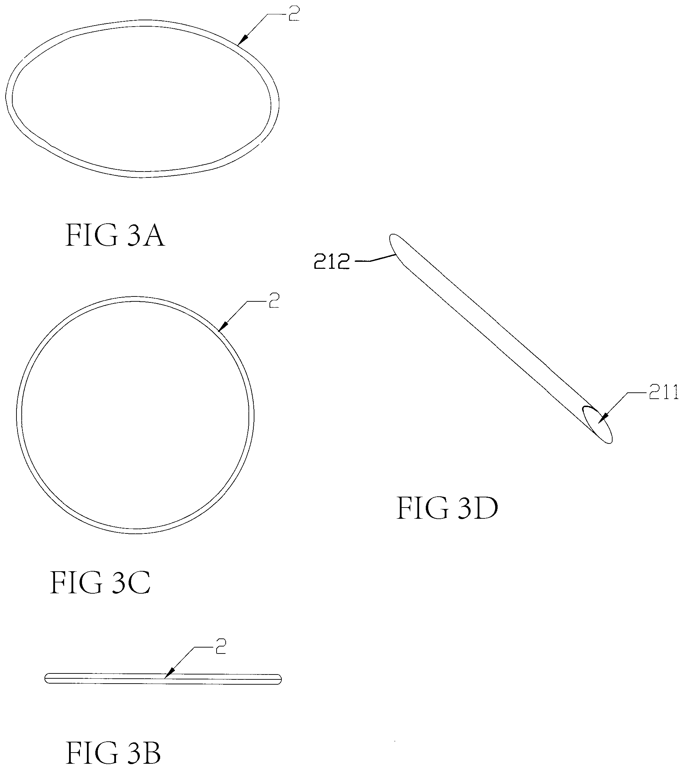

Figs 3A, 3B, and 3C are illustrations showing various views of the cushion ring shown in Fig 1A in an embodiment where cushion ring is circular;
Fig 3D is an illustration of a linear cushion ring used in one embodiment;

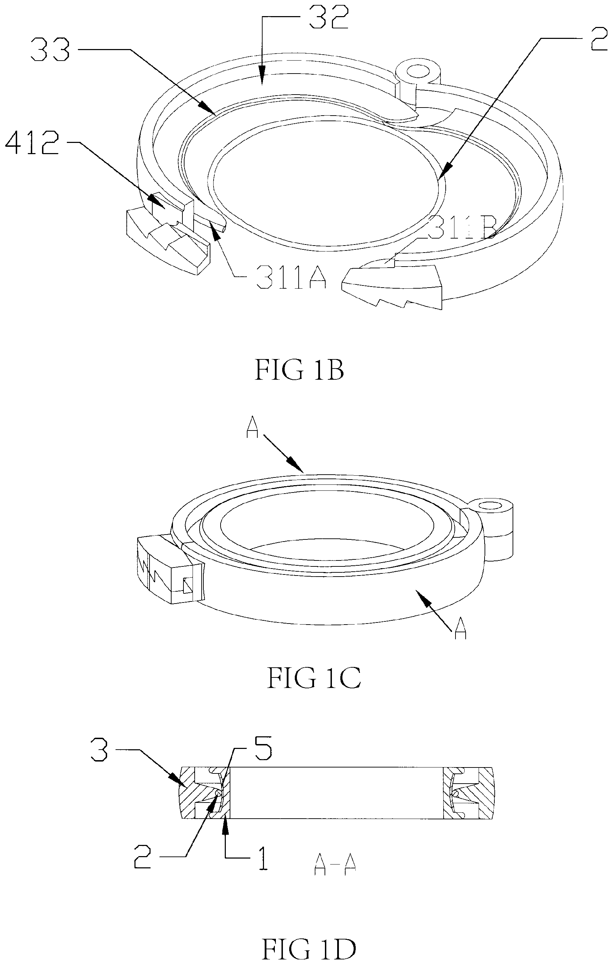

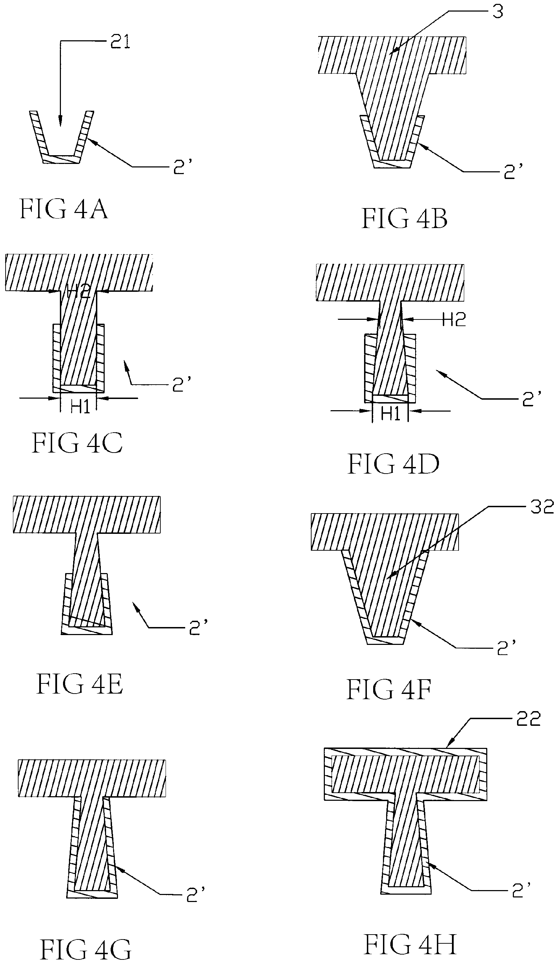

Figs 4A, 4B, 4C, 4D, 4E, 4F, 4G, and 4H illustrate various embodiments of how the cushion ring and blade shown in Fig 1 may be joined;

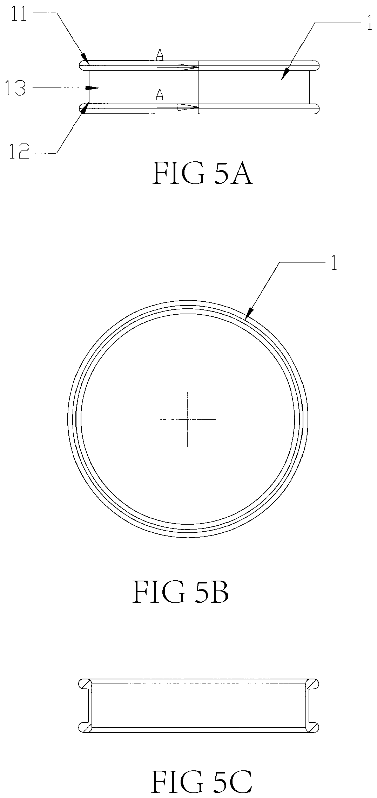

Figs 5A, 5B, and 5C illustrate three different views of the balanus ferrule of Fig 1A;

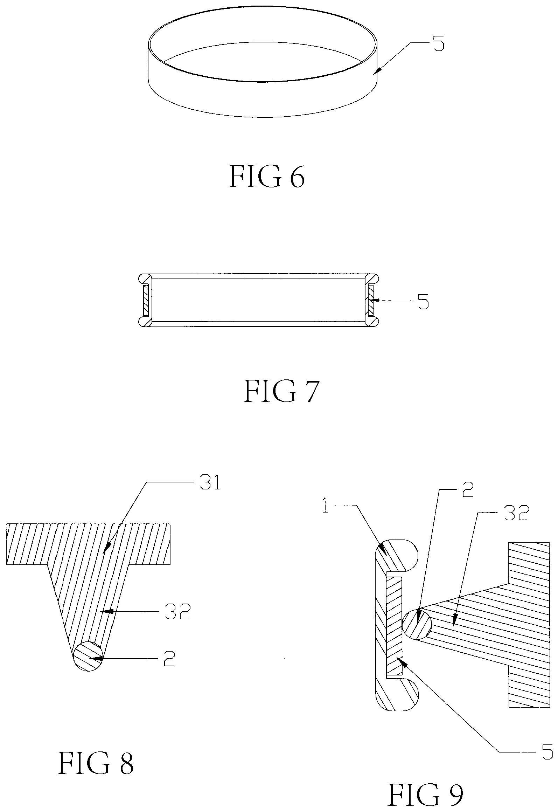

Fig 6 illustrates a circular elastic pad used in one embodiment;
Fig 7 illustrates a cross-section of the balanus ferrule used with the circular elastic pad;
Fig 8 illustrates a cross-section of the cushion ring as it is seated into the half-ring groove of the fixture device;
Fig 9 illustrates a cross-section of the fixture device, cushion ring, balanus ferrule, and circular elastic pad in one embodiment as they are interconnected with each other;

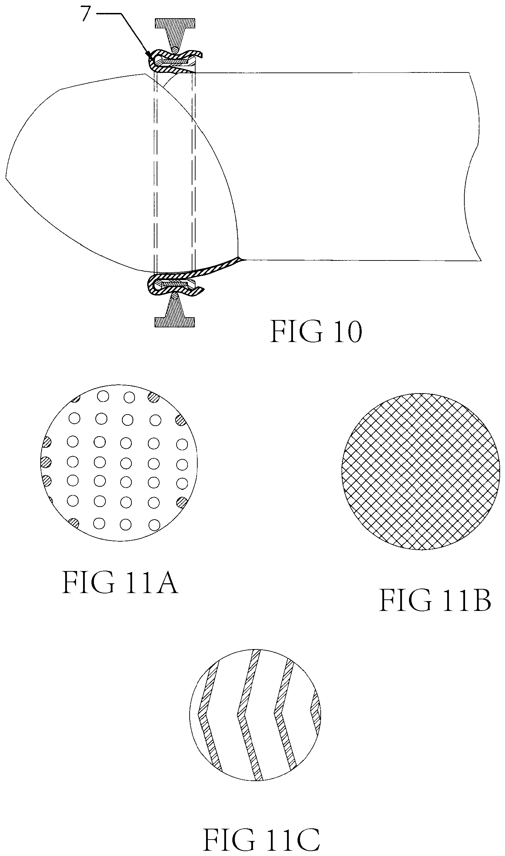

Fig 10 illustrates one embodiment of an apparatus for circumcising a penis at it is being used during a circumcision procedure;
Figs 11A, 11B, and 11C illustrate three embodiments of micro mechanisms that prevent skin adhesion on the cushion ring and circular elastic pad;

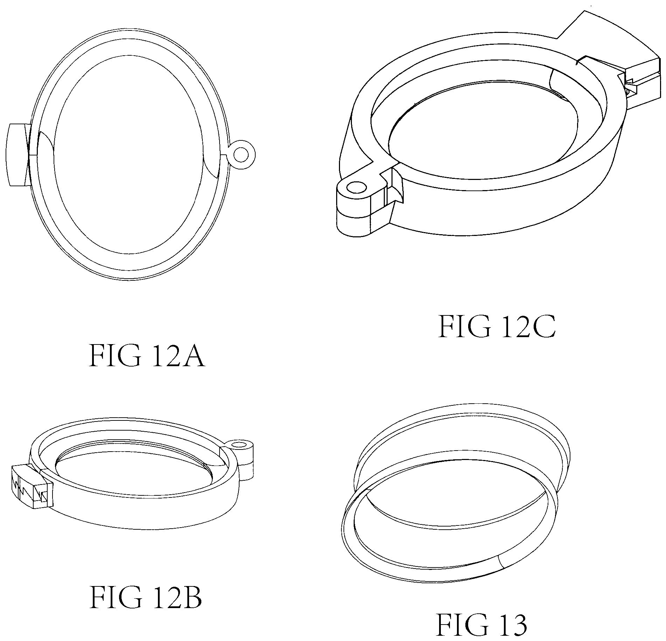

Figs 12A and 12B illustrate various views of the fixture device in an embodiment where the fixture device is elliptical in nature;
Fig 12C illustrate another embodiment of the fixture device in an embodiment where the fixture device is elliptical and oblique;
Figs 13 illustrate another embodiment of the balanus ferrule, shown as an elliptical and oblique structure;

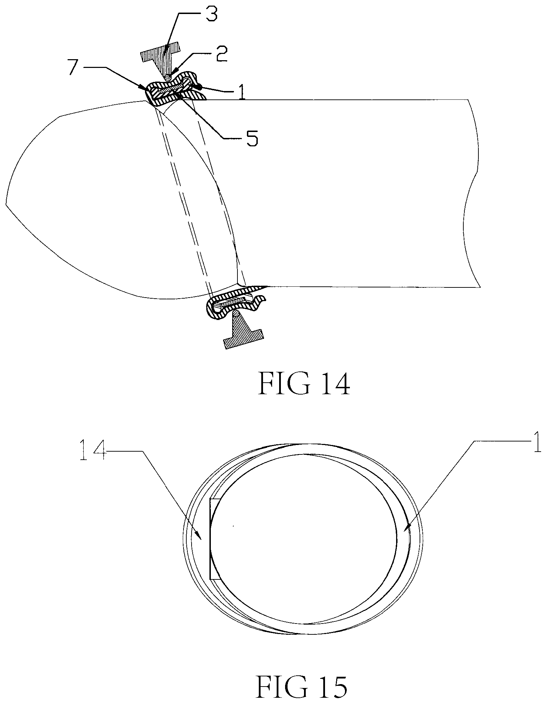

Fig 14 is an illustration of the elliptical and oblique embodiment of the apparatus for circumcision as shown in Figs 12C, 13 as it is used during circumcision surgery;
Fig 15 illustrates a balanus ferrule in one embodiment, comprising a notch for containing the frenulum of prepuce;

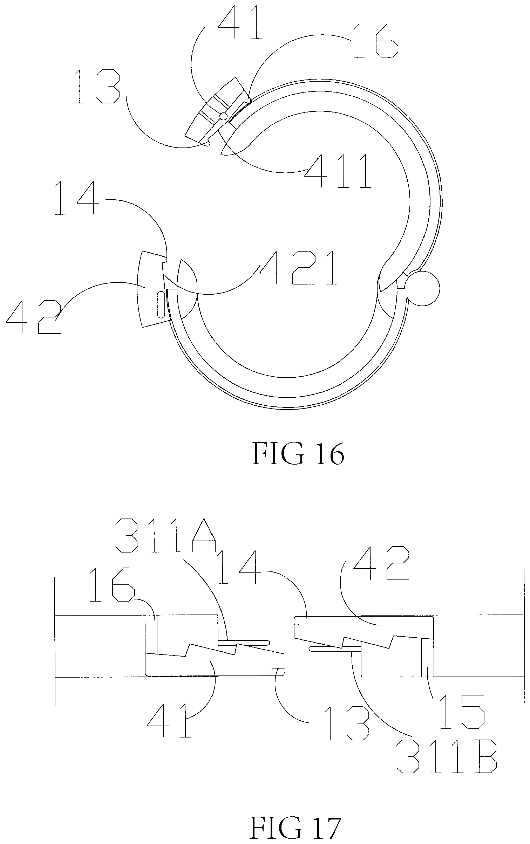

Figs 16 and 17 are illustrations of the fixture device in an embodiment where the fixture device comprises a hook and a linear groove;

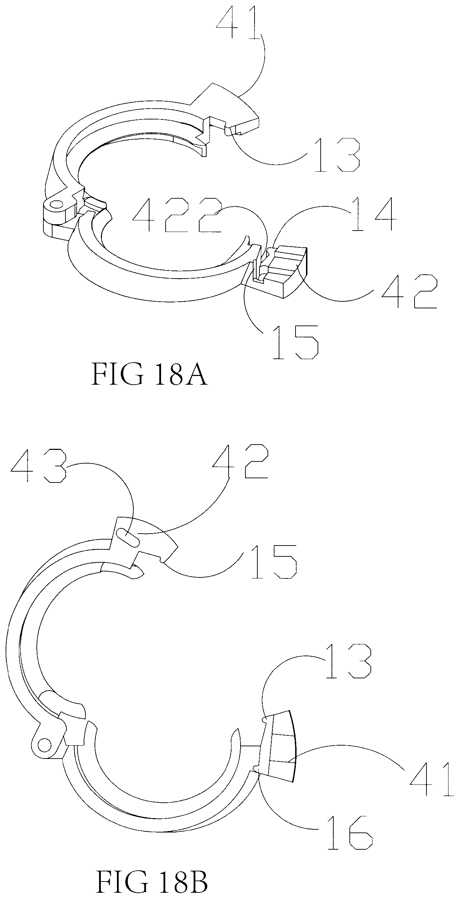

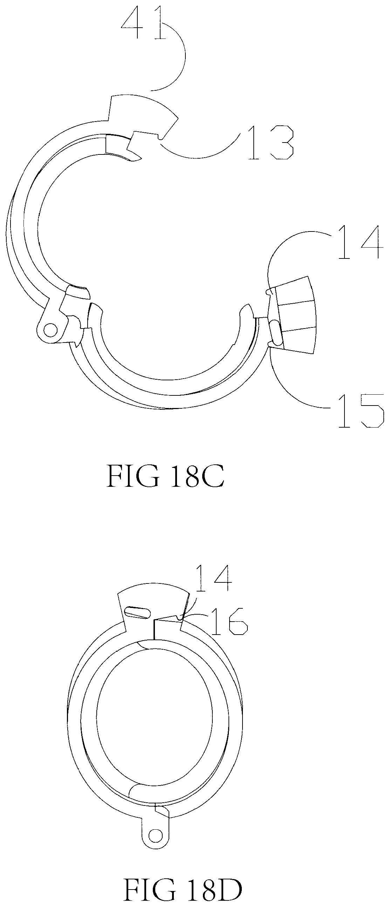

Figs 18A, 18B, 18C, and 18D are illustrations of various embodiments of the apparatus for circumcising a penis in an elliptical and oblique shape, comprising a hook and a linear groove;

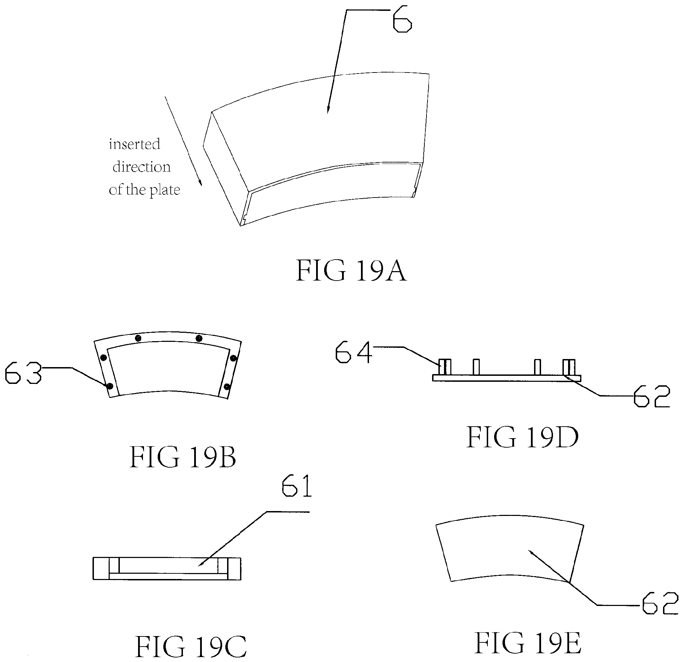

Figs 19A, 19B, 19C, 19D, and 19E illustrate a protective cover used in conjunction with the fixture device, with Fig 19A illustrating a perspective view of the protective cover body, Fig 19B illustrating a bottom view of the protective cover body, Fig 19C illustrating a cut-away view of the interior of the protective cover body, Fig 19D illustrating a side view of a protective cover cap used to seal the protective cover body, and Fig 19E illustrating a bottom view of the protective cover cap;

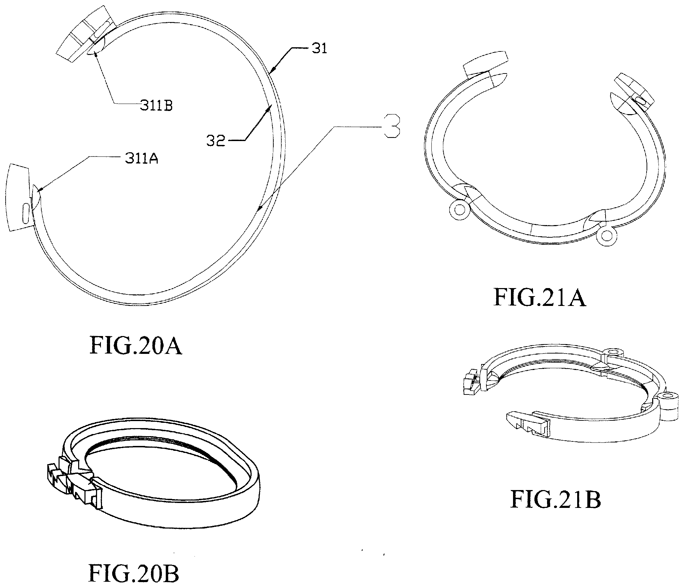

Figs 20A and 20B illustrate an embodiment of a fixture device shown as a flexible, C-shaped structure; and
Figs 21A and 21B illustrate an embodiment of a fixture device comprising three sections.

### Detailed Description of the Preferred Embodiments of the Invention

As shown in Fig 1A, a disposable apparatus for circumcising a penis (circumcision apparatus) comprises fixture device 3, balanus ferrule 1, the fixture device 3 comprising surrounding wall 31 and blade 32. The fixture device 3 forms an opening through which a penis can be easily placed during a circumcision procedure. The fixture device comprises at least one hinged connection, allowing the fixture device to close around the penis, where it may then be secured by a buckling device 4 incorporated into the fixture device structure.

In order to avoid the pain resulting from clamping fixture device 3 and balanus ferrule 1 onto the prepuce, the present invention utilizes a cushion ring 2 that is provided on fixture device 3. As shown in Fig 1B, a cushion ring 2 is connected to the inner side of the blade 32 (which is closest to the balanus ferrule 1 during a circumcision procedure).

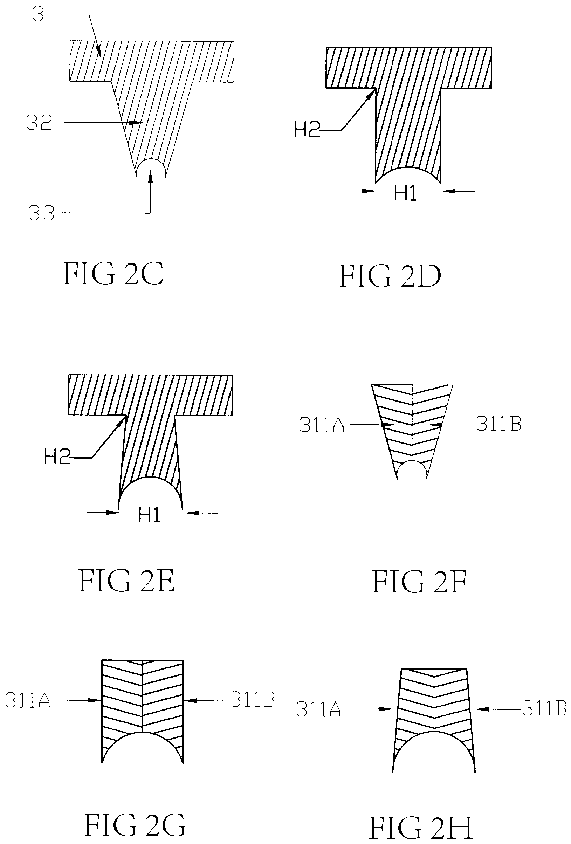

According to a first embodiment, the groove 33 is formed on the inside circumference of the blade 32. As shown in Fig 2C, in one embodiment, the cross-section of groove 33 is of semi-circular shape. As shown in Figs 3A, 3B, and 3C, the cross-section of the cushion ring interconnected with groove 33 is of circular shape, and the diameter thereof is less than or equal to that of the circular groove. The circumference of the cushion ring is about equal to that of the blade edge when the fixture device is closed. The cushion ring is typically held in place by the geometry of the groove and ring. Of course, the groove 33 may, alternatively, comprise an arc, semi-elliptic, square, or triangular shape, any of which can be made to accept the cushion ring 2. As shown in Figs 2D and 2E, in order to save materials, the inner side of the blade (H1) can be equal to or thicker than that of outer side of the blade (H2).

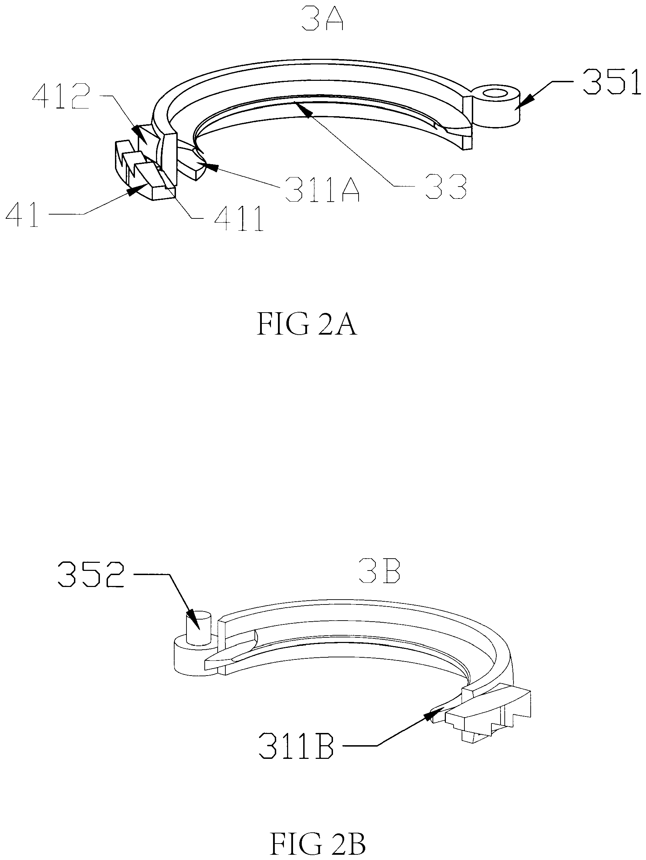

As shown in Figs. 1B, 2A and 2B, the blade of the left semi-ferrule 3A comprises a first blade mating portion 311A and the blade of the right semi-ferrule 3B comprises a second blade mating portion 311B at the open end of the blade, respectively. These portions of the blade are designed to slide over each other, or overlap, to form a complete blade structure (e.g., ring) as the fixture device is placed into a closed position. Thus, the thickness of both the first and second blade mating portions are thinner than the majority of the blade structures. In one embodiment, the mating portions comprise rounded edges. Figs 2F, 2G, and 2H illustrate three embodiments of the first and second mating blade portions when the fixture device is in a closed position. The groove formed on the interior circumference of the blade extends into the mating portions, however the cross section of the groove in the mating portions are half of what they are in the majority of the blade. Thus, when the fixture device is placed into a closed position, the groove that is formed on the majority of the blade continues uninterrupted in the mating portions, as the groove is completed as the mating portions align with one another.

As shown in Figs. 1A, 1B, 2A and 2B, the fixture device 3 comprises a two-section design, each section having an open end and a connecting end. In this embodiment, fixture device 3 comprises the left semi-ferrule 3A and right semi-ferrule 3B. The connecting ends are rotatably connected to one another via a hinged connection 35, which comprises connecting linear groove 351 on one semi-ferrule and connecting rod shaft 352 on the other semi-ferrule. The open end on left semi-ferrule 3A comprises a means for mating with a reciprocal means located on the open end of the right semi-ferrule 3B. In this way, the fixture device can be easily applied and removed during circumcision surgery. Furthermore, adequate rigidity of the blade may be achieved by using hard materials. The design of the first and second mating blade portions effectively prevent the blade from pinching the penis when using the circumcision device during surgery. Additionally, the structure effectively guides the cushion ring into the blade groove. Thus, the cushion ring fits easily within the complete groove formed as the fixture device is closed, and is not clamped or pinched at the open or connecting ends.

In another embodiment, each semi-ferrule may comprise two or more blade. As shown in Fig 2I and 2J (depicting a cross section of either right blade 32 or left blade), each semi-ferrule comprises two blade, each comprising an inner circumference having a groove to accommodate a cushion ring, while a cavity 34 is formed between the two blade for containing cotton wool or ointment. As shown in Fig 2K illustrating the mating portions for open or connecting end for both blade, the left semi-ferrule comprises two mating blade portions 311A (inner), and the right semi-ferrule comprises two mating blade portions 311B (outer). As the circumcision device is closed, the two mating blade portions 311B overlap the two mating blade portions 311A to form a complete ring structure.

Besides the closed circular structure shown in Fig 3A. In an alternative embodiment, the cushion ring 2 may comprise the linear structure shown in Fig 3D, which can be directly inserted into groove 33 of the blade. In this embodiment, each end 211 and 212 of the linear structure are cut off at an angle, such as 45 degrees, and these ends lie on top of the first blade mating portion and second blade mating portion, respectively, after it has been inserted into the groove. When the fixture device is closed, the linear cushion ring forms a full ring shape.

Another embodiment of the cushion ring is illustrated in Figs 4A and 4B. In this embodiment, the cushion ring 2' comprises a groove 21 that overlaps the inner circumference of one or both blade. As shown in Figs 4F, 4G and 4H, groove 21 may alternatively overlap a portion of or an entire blade.

Traditionally, the thickness of the inner circumference of a blade (H1) is thinner than the outer circumference (H2) thickness. Alternatively, the width (H1) can be equal to or larger than H2. This design can reduce the amount of materials needed to manufacture the blade/cushion ring. Another advantage is that it may be desirable for the cushion ring 2 to be attached to the blade. Fig 4C illustrates that width H1 is equal to width H2, while Figs 4D and 4E illustrate that width H1 is greater than width H2. The method in which H1 is greater than or equal to H2 is desirable so that the cushion ring 2 may be steadily affixed to the blade.

In one embodiment, the cushion ring 2' is manufactured from medical non-toxic silica-gel and may be directly adhered to the blade inner circumference. In other embodiments, the cushion ring 2 may be manufactured from virtually any combination of plastics and silica-gel. In yet sill other embodiments, the blade are manufactured from a relatively hard material, such as plastic, while the cushion ring is manufactured with a less-hard substance, such as silica-gel, so that the cushion ring fits adheres to the groove by virtue of an integral formation between the plastic and the silica-gel. Furthermore, the surrounding wall 31 may be coated with a silica-gel layer 22 that adheres to the surrounding wall 31 (i.e., fixture device) through a single integral formation between plastics and silica-gel. In one embodiment, the entire fixture device is coated with a silica-gel layer, enabling the penis to directly contact this soft surface, thereby effectively increasing the patient's comfort.

Figs 5A, 5B, and 5C illustrate three views of balanus ferrule 1 shown in Fig. 1A. A first rim 11 and a second rim 12 create a circular cavity 13 between the first rim 11 and the second rim 12. As shown in Fig 6, a circular elastic pad 5 comprises a ring made of an elastic material, such as rubber or other stretchable synthetic material.

As shown in Fig 7, when in use, the elastic pad 5 is stretched over one of the rims (either rim 11 or rim 12) of the balanus ferrule 1, where it then fits snugly around the circular cavity.

Fig 8 illustrates a cross section of one of the blade and cushion ring inserted into a groove formed along an interior circumference of the blade. Fig 9 illustrates a cross section of the blade/ring combination of Fig 8 in contact with a cross section of the elastic pad 5 placed around the balanus ferrule 1. During a circumcision operation, a penis is placed through the balanus ferrule 1 having the circular elastic pad 5 in place over its circular cavity. Next, the prepuce 7 is unfurled to cover the balanus ferrule 1 with the unfurled portion of the prepuce extending from the ferrule. Then, the circular cushion ring 2 is placed around the exterior surface of the prepuce. Next, the fixture device 3, in an open position, is placed around the cushion ring 2, aligning the groove in each blade with the cushion ring 2. Finally, the fixing device is closed by bringing the two open ends of each semi-ferrule together and locking the fixing device in a closed position using the buckling device. The result, shown in a cross-sectional view, is shown in Fig 10. Using this technique, the first and second mating blade portions can effectively prevent the skin on the penis from being pinched at the open and connecting ends of the fixture device and, additionally, the circular cushion ring is easily guided into the blade groove. Both the circular cushion ring and surrounding elastic pad are typically made of soft, non-toxic medical silica-gel, providing a certain degree of expansion and effectively minimizing the pain and discomfort normally associated with circumcision surgery.

Turning back to Fig 1C, the outer circumference of the surrounding wall of the fixture device comprises a beautiful arc shape. As show in 1D, the exterior circumference of the balanus ferrule comprises a curved profile which can prevent the blade sliding from the middle to either rim of the balanus ferrule.

In order to prevent skin adhesion during removal of the apparatus from the penis after surgery, a micro mechanism that prevents skin adhesion may be provided on the surface of either the circular cushion ring 2, circular elastic pad 5, or both. The micro mechanism may comprise depressions or raised portions, such as the sinking and/or bulging dots shown in Fig 11A, the crossed bulging and/or sinking streaks shown in Fig 11B, or the grooves and/or protrusions shown in Fig 11C. Due to the existence of the micro mechanisms, removal of the circumcision apparatus reduces the occurrence of skin adhesion, making the process of removing the circumcision apparatus easier.

In an embodiment shown in Figs 12A and 12B, the fixture device 3 and balanus ferrule 1 comprise an elliptic structure. This structure may more closely resemble the cross section of an actual penis, especially the shape of the coronal sulcus. As shown in Figs 12C and 13, the fixture device and the balanus ferrule are elliptical and oblique. As shown in Fig 14, the circumcision apparatus closely matches the oblique shape of coronal sulcus, providing more comfort to the patient and ease of operation for the surgeon.

As shown in Fig 15, a notch 14 is formed for accommodating the frenulum of prepuce is provided at one side of the first and second edges of the balanus ferrule 1. While in use, the balanus ferrule may be rotated so that notch 14 accommodates the frenulum of prepuce. The notch 14 advantageously protects the frenulum and allows the circumcision device to more closely fit a human penis. Thus, the pain resulting from chaffing of the frenulum during a nocturnal erection may be reduced, thus increasing patient comfort.

The fixture device is closed (only closed not opened to avoid reuse of the apparatus) by the use of a buckling device. The buckling device may comprise any number of structures known in the art, such as a screw located on an open end of one of the semi-ferrules and a threaded linear groove at the other open end on the other semi-ferrule that comprise fixture device 3. In another embodiment, the buckling device 4 comprises the detent block structure shown in Figs 16, 17. As shown in Fig 17, the buckling device comprises an upper scalariform detent block 41 and a lower scalariform detent block 42 that are arranged in a "staggered" formation. The first mating blade portion 311A lies in a plane slightly above the lower scalariform detent block 41 and the second mating blade portion 311B lies in a plane slightly under the upper scalariform detent block 42, creating a fixture device that is self-contained and capable of being opened and closed using the buckling device.

In order to further avoid accidental opening of the buckling device, the aforesaid upper scalariform detent block 41 and lower scalariform detent block 42 may comprise a hook and linear groove that corresponds with each other. As shown in Figs 16, 17, in one embodiment, an upper hook 13 may be provide at the outer end of upper scalariform detent block 41 and a lower linear groove 16 is provided at the inner end of the upper scalariform detent block 41, while a lower hook 14 is provide at the outer end of lower scalariform detent block 42 and an upper linear groove 15 is provided at the inner end of the lower scalariform detent block 42. To close the fixture device, the upper hook 13 interacts with the upper linear groove 15, while the lower hook 14 interacts with the lower linear groove 16. When the an upper scalariform detent block 41 and a lower scalariform detent block 42 are engaged with each other, the hook and linear groove further assures that the fixture device will remain closed.

In view of the interaction between the hooks and linear grooves in the above-described embodiment, the inner sides of the upper scalariform detent block 41 and lower scalariform detent block 42 may comprise incline surfaces, and incline projecting portions, located on the surrounding walls, the incline projecting portions substantially matching the incline surfaces. This feature is shown, illustrating a first incline surface 411 and a first incline projecting portion 412 on the upper scalariform detent block 41 and a second incline surface 421 and second incline projecting portion 422 on the lower scalariform detent block 42. The upper hook 13 is provided at the outer side of the first incline surface 411 and the lower hook 14 is provided at the outer side of second incline surface 421. The lower linear groove 16 is provided at the end of the first incline projecting portion 412 and the upper linear groove 15 at the end of the second incline projecting portion 422. There may be holes which substantially matching the hooks. The hooks and linear grooves or holes further substantially match with the upper and lower tooth-like detent blocks, ensuring effective vertical and horizontal locking with each other, and preventing accidental openings of the fixture device 3.

As shown in Fig 18B, 18C, and 18D an unlocking hole 43 may be provided on the lower scalariform detent block 42 or on the upper scalariform detent block 41. When a circumcision procedure has been completed, a special tool may be inserted into the unlocking linear groove 43, thereby prying the detent blocks from one another and allowing removal of the circumcision apparatus.

Fig 19A and 19E illustrate a protective cover 6 that may be placed onto the buckling device to prevent accidental opening of the fixture device after surgery. The protective cover 6 comprises a cover body having a bottom plate, two side walls and a top wall. Cap 62 substantially matches the bottom plate so that protective cover 6 forms a cavity 61 that substantially matches the contour of the buckling device when cap 62 is placed on top of the cover body. The cap 62 is secured to the cover body typically using one or more fastening devices, such as screws, bolts, Velcro, etc., or using one or more rods 64 attached to cap 62, that may be snugly inserted into corresponding linear grooves 63 formed on the cover body rim. The protective cover may be constructed of, in one embodiment, resilient silica-gel for convenient use. The protective cover can effectively minimize the risk of accidental openings of the fixture device after surgery, which may result from an erection of the penis, thereby increasing the likelihood of a successful operation.

Fig 20 illustrates another embodiment of the fixture device, as fixture device 30. Fixture device 30 comprises a C-shaped ring that is made of soft materials that may enable it to open wider when needed.

Figs 21A and 21B illustrate an embodiment where the fixture device comprises three sections. The three-section design effectively eliminates the single hinge from the center of the circumcision apparatus and replaces it with the two hinges shown in Figs 21A and 21B. Thus, the placement of the two hinges allow the scrotum to be placed therebetween, thereby reducing friction with the scrotum.

## Claims

1. A disposable circumcision apparatus, comprising:
a fixture device (3) comprising a surrounding wall (31), the surrounding wall comprising an opening;
a blade (32) formed along an interior surface of the surrounding wall, the blade comprising a first blade mating portion (311A) and a second blade mating portion (311B) that mate with each other when the fixture device is in a closed position, the blade further comprising a blade interior circumferential edge;
means (41, 42) for maintaining the fixture device in a closed position; and
a balanus ferrule (1);
**characterised by** a cushion ring (2, 2') for being positioned against the blade, the balanus ferrule being arranged for placement within the cushion ring and fixture device, so that in use a prepuce (7) may be interposed between the cushion ring and the balanus ferrule and the cushion ring interposed between the blade and the prepuce; and
a groove (21, 33), wherein the groove (33) is formed along the blade (32) interior circumferential edge for receiving the cushion ring (2), or is formed (21) along a circumference of the cushion ring (2') for receiving the blade (32) interior circumferential edge or for receiving the entire blade (32).

2. An apparatus as claimed in claim 1, wherein:
the groove (33) is formed along the blade interior circumferential edge for receiving the cushion ring (2).

3. An apparatus as claimed in claim 1, wherein:
the groove (21) is formed along a circumference of the cushion ring (2') for receiving the blade interior circumferential edge.

4. An apparatus as claimed in claim 1, wherein:
the groove (21) is formed along a circumference of the cushion ring (2') and the entire blade is placed within the groove.

5. An apparatus as claimed in claims 1 or 3 or 4, wherein:
the cushion ring (2, 2') is made of silica-gel and the blade (32) is made of plastic, and the cushion ring adheres to the blade through an integral formation between plastics and silica-gel.

6. An apparatus as claimed in claim 5, wherein:
the fixture device (3) is coated with a silica-gel layer (22), and the silica-gel layer adheres to the fixture device through an integral formation between plastics and silica-gel.

7. An apparatus as claimed in claim 1 or 2 or 3 or 4, wherein:
the blade interior circumferential edge thickness (H1) is greater than or equal to the thickness (H2) of exterior circumferential edge of the blade where the blade contacts an interior circumferential surface of the fixture device.

8. An apparatus as claimed in claim 1 or 2 or 3 or 4, further comprising:
a circular elastic pad (5), wherein the elastic pad is placed around the exterior of the balanus ferrule (1).

9. An apparatus as claimed in claim 8, wherein the balanus ferrule (1) further comprises:
a first rim (11) located on a first side of the balanus ferrule; and
a second rim (12) located on a second side of the balanus ferrule; wherein
the first and second rims forma ring-shaped cavity (13) upon which the elastic pad (5) rests.

10. The apparatus of claim 9, wherein the cushion ring and the elastic pad comprises a micro mechanism for preventing skin adhesion to the cushion ring and the elastic pad.

11. An apparatus as claimed in claim 10, wherein:
the micro mechanism is selected from the group consisting of sinking and bulging dots, crossed sinking and bulging lines, grooves, protrusions, and an uneven structure.

12. An apparatus as claimed in claim 1, wherein:
the fixture device (3) and the balanus ferrule (1) comprise an elliptic or obliquely elliptic structure corresponding to a cross section of a coronal sulcus.

13. An apparatus as claimed in claim 1, wherein the balanus ferrule (1) further comprises a notch (14) for accommodating a frenulum of prepuce.

14. An apparatus as claimed in claim 1 or 12, wherein:
the fixture device (3) is C-shaped.

15. An apparatus as claimed in claim 1 or 12, wherein:
the fixture device comprises three sections connected together at two hinge points, the hinge points staggered from each other so that they minimize contact with a scrotum during circumcision surgery.

16. An apparatus as claimed in claim 1, wherein the means for maintaining the fixture device in a closed position comprises upper and lower scalariform detent blocks (41, 42), each of the scalariform detent blocks comprising a hook (13, 14) and a hole, wherein the hooks are sized and shaped to fit within the holes, respectively.

17. An apparatus as claimed in claim 1, wherein:
the means for maintaining the fixture device in a closed position comprises a screw on one end of the fixture device and a linear threaded hole on the other end of the fixture device.

18. An apparatus as claimed in claim 1 or 16, further comprising a protective cover (6) sized and shaped to fit over the means for maintaining the fixture device in a closed position.

19. An apparatus as claimed in claim 2, wherein:
the cross-section of the groove (33) is selected from the group consisting of an arc, a semi-circle, a semi-ellipse, and a triangle; and
the cross-section of the first blade mating portion (311A) and the second blade mating portion (311B) combined is selected from the group consisting of an arc, a semi-circle, a semi-ellipse, and a triangle; and
the cushion ring (2) comprising a cross section substantially matching the cross section of the groove (33).

20. An apparatus as claimed in claim 2, wherein:
the cushion ring (2) is of ring shape and the circumference thereof corresponds with that of the blade (32).

21. An apparatus as claimed in claim 1, 3 or 4, wherein:
the fixture device (3) comprises one, two, or three blades (32).

22. An apparatus as claimed in claim 1, 3 or 4, wherein:
round angles are provided at the first and second blade mating portions (311A, 311B).

## Patentansprüche

1. Einweg-Beschneidungsvorrichtung, die Folgendes umfasst:
eine Einspannanordnung (3), die eine Umgebungswand (31) umfasst, wobei die Umgebungswand eine Öffnung umfasst;
eine Klinge (32), die entlang einer Innenfläche der Umgebungswand ausgebildet ist, wobei die Klinge einen ersten Klingenpassteil (311A) und einen zweiten Klingenpassteil (311B) umfasst, die miteinander zusammenpassen, wenn die Einspannanordnung in einer geschlossenen Position ist, wobei die Klinge weiterhin eine Klingeninnenumfangskante umfasst;
Mittel (41, 42) zum Halten der Einspannanordnung in einer geschlossenen Position und
eine Eichelhülse (1);
**gekennzeichnet durch** einen Dämpfungsring (2, 2'), um gegen die Klinge positioniert zu werden, wobei die Eichelhülse zur Platzierung in dem Dämpfungsring und der Einspannanordnung eingerichtet ist, so dass eine Vorhaut (7) im Gebrauch zwischen dem Dämpfungsring und der Eichelhülse eingeschoben werden kann und der Dämpfungsring zwischen der Klinge und der Vorhaut eingeschoben werden kann; und
eine Nut (21, 33), wobei die Nut (33) entlang der Innenumfangskante der Klinge (32) zum Aufnehmen des Dämpfungsrings (2) ausgebildet ist oder entlang einem Umfang des Dämpfungsrings (2') zum Aufnehmen der Innenumfangskante der Klinge (32) oder zum Aufnehmen der gesamten Klinge (32) ausgebildet ist (21).

2. Vorrichtung nach Anspruch 1, wobei:
die Nut (33) entlang der Klingeninnenumfangskante zum Aufnehmen des Dämpfungsrings (2) ausgebildet ist.

3. Vorrichtung nach Anspruch 1, wobei:
die Nut (21) entlang einem Umfang des Dämpfungsrings (2') zum Aufnehmen der Klingeninnenumfangskante ausgebildet ist.

4. Vorrichtung nach Anspruch 1, wobei:
die Nut (21) entlang einem Umfang des Dämpfungsrings (2') ausgebildet ist und die gesamte Klinge in der Nut platziert ist.

5. Vorrichtung nach den Ansprüchen 1 oder 3 oder 4, wobei:
der Dämpfungsring (2, 2') aus Kieselgel hergestellt ist und die Klinge (32) aus Kunststoff hergestellt ist und
der Dämpfungsring durch eine integrale Ausbildung zwischen Kunststoffen und Kieselgel an der Klinge haftet.

6. Vorrichtung nach Anspruch 5, wobei:
die Einspannanordnung (3) mit einer Kieselgelschicht (22) beschichtet ist und die Kieselgelschicht durch eine integrale Ausbildung zwischen Kunststoffen und Kieselgel an der Einspannanordnung haftet.

7. Vorrichtung nach Anspruch 1 oder 2 oder 3 oder 4, wobei:
die Klingeninnenumfangskantendicke (H1) größer gleich der Dicke (H2) einer Außenumfangskante der Klinge ist, wo die Kline eine Innenumfangsfläche der Einspannanordnung berührt.

8. Vorrichtung nach Anspruch 1 oder 2 oder 3 oder 4, die weiterhin Folgendes umfasst:
ein kreisförmiges elastisches Polster (5), wobei das elastische Polster um die Außenseite der Eichelhülse (1) platziert ist.

9. Vorrichtung nach Anspruch 8, wobei die Eichelhülse (1) weiterhin Folgendes umfasst:
einen ersten Rand (11), der sich auf einer ersten Seite der Eichelhülse befindet; und
einen zweiten Rand (12), der sich auf einer zweiten Seite der Eichelhülse befindet; wobei
der erste und der zweite Rand eine ringförmige Aussparung (13) bilden, auf der das elastische Polster (5) ruht.

10. Vorrichtung nach Anspruch 9, wobei der Dämpfungsring und das elastische Polster einen Mikromechanismus zum Verhindern einer Hautanhaftung an dem Dämpfungsring und dem elastischen Polster umfassen.

11. Vorrichtung nach Anspruch 10, wobei:
der Mikromechanismus aus der Gruppe bestehend aus abgesenkten und vorgewölbten Punkten, sich kreuzenden abgesenkten und vorgewölbten Linien, Nuten, Vorsprüngen und einer unebenen Struktur ausgewählt ist.

12. Vorrichtung nach Anspruch 1, wobei:
die Einspannanordnung (3) und die Eichelhülse (1) eine elliptische oder schräg-elliptische Struktur umfassen,
die einem Querschnitt einer Penisfurche entspricht.

13. Vorrichtung nach Anspruch 1, wobei die Eichelhülse (1) weiterhin eine Kerbe (14) zum Unterbringen eines Vorhautbändchens umfasst.

14. Vorrichtung nach Anspruch 1 oder 12, wobei:
die Einspannanordnung (3) C-förmig ist.

15. Vorrichtung nach Anspruch 1 oder 12, wobei:
die Einspannanordnung drei Abschnitte umfasst, die an zwei Scharnierpunkten miteinander verbunden sind, wobei die Scharnierpunkte voneinander versetzt sind, so dass sie eine Berührung mit einem Hodensack während eines chirurgischen Beschneidungseingriffs minimieren.

16. Vorrichtung nach Anspruch 1, wobei das Mittel zum Halten der Einspannanordnung in einer geschlossenen Position einen oberen und einen unteren leiterförmigen Arretierungsblock (41, 42) umfasst, wobei jeder der leiterförmigen Arretierungsblöcke einen Haken (13, 14) und ein Loch umfasst, wobei die Haken so bemessen und geformt sind, dass sie jeweils in die Löcher passen.

17. Vorrichtung nach Anspruch 1, wobei:
das Mittel zum Halten der Einspannanordnung in einer geschlossenen Position eine Schraube an einem Ende der Einspannanordnung und ein lineares Gewindeloch an dem anderen Ende der Einspannanordnung umfasst.

18. Vorrichtung nach Anspruch 1 oder 16, die weiterhin eine Schutzabdeckung (6) umfasst, die so bemessen und geformt ist, dass sie über das Mittel zum Halten der Einspannanordnung in einer geschlossenen Position passt.

19. Vorrichtung nach Anspruch 2, wobei:
der Querschnitt der Nut (33) aus der Gruppe bestehend aus einem Kreisbogen, einem Halbkreis, einer Halbellipse und einem Dreieck ausgewählt ist und
der Querschnitt der Kombination des ersten Klingenpassteils (311A) und des zweiten Klingenpassteils (311B) aus der Gruppe bestehend aus einem Kreisbogen, einem Halbkreis, einer Halbellipse und einem Dreieck ausgewählt ist und
der Dämpfungsring (2) einen Querschnitt umfasst, der im Wesentlichen mit dem Querschnitt der Nut (33) übereinstimmt.

20. Vorrichtung nach Anspruch 2, wobei:
der Dämpfungsring (2) eine Ringform aufweist und der Umfang dieses dem der Klinge (32) entspricht.

21. Vorrichtung nach Anspruch 1, 3 oder 4, wobei:
die Einspannanordnung (3) eine, zwei oder drei Klingen (32) umfasst.

22. Vorrichtung nach Anspruch 1, 3 oder 4, wobei:
Vollwinkel an dem ersten und dem zweiten Klingenpassteil (311A, 311B) vorgesehen sind.

## Revendications

1. Appareil de circoncision jetable, comportant :
un dispositif de fixation (3) comportant une paroi périphérique (31), la paroi périphérique comportant une ouverture ;
une lame (32) formée le long d'une surface intérieure de la paroi périphérique, la lame comportant une première partie d'accouplement de lame (311A) et une deuxième partie d'accouplement de lame (311B) qui s'accouplent l'une par rapport à l'autre quand le dispositif de fixation est dans une position fermée, la lame comportant par ailleurs un bord circonférentiel intérieur de lame ;
un moyen (41, 42) de maintien du dispositif de fixation dans une position fermée ; et
une bague de gland (1) ;
**caractérisé par** un anneau à coussinet (2, 2') à des fins de positionnement contre la lame, la bague de gland étant agencée à des fins de mise en place à l'intérieur de l'anneau à coussinet et du dispositif de fixation, de telle sorte que, lors de l'utilisation, un prépuce (7) peut être intercalé entre l'anneau à coussinet et la bague de gland et l'anneau à coussinet peut être intercalé entre la lame et le prépuce ; et
une rainure (21, 33), la rainure (33) étant formée le long du bord circonférentiel intérieur de la lame (32) à des fins de réception de l'anneau à coussinet (2), ou étant formée (21) le long d'une circonférence de l'anneau à coussinet (2') à des fins de réception du bord circonférentiel intérieur de la lame (32) ou à des fins de réception de la totalité de la lame (32).

2. Appareil selon la revendication 1, dans lequel :
la rainure (33) est formée le long du bord circonférentiel intérieur de la lame à des fins de réception de l'anneau à coussinet (2).

3. Appareil selon la revendication 1, dans lequel :
la rainure (21) est formée le long d'une circonférence de l'anneau à coussinet (2') à des fins de réception du bord circonférentiel intérieur de la lame.

4. Appareil selon la revendication 1, dans lequel :
la rainure (21) est formée le long d'une circonférence de l'anneau à coussinet (2') et la totalité de la lame est placée à l'intérieur de la rainure.

5. Appareil selon les revendications 1 ou 3 ou 4, dans lequel :
l'anneau à coussinet (2, 2') est réalisé en gel de silice et la lame (32) est réalisée en plastique, et l'anneau à coussinet adhère à la lame par une formation solidaire entre le plastique et le gel de silice.

6. Appareil selon la revendication 5, dans lequel :
le dispositif de fixation (3) est revêtu au moyen d'une couche de gel de silice (22), et la couche de gel de silice adhère au dispositif de fixation par une formation solidaire entre le plastique et le gel de silice.

7. Appareil selon les revendications 1 ou 2 ou 3 ou 4, dans lequel :
l'épaisseur (H1) du bord circonférentiel intérieur de la lame est supérieure ou égale à l'épaisseur (H2) du bord circonférentiel extérieur de la lame là où la lame entre en contact avec une surface circonférentielle intérieure du dispositif de fixation.

8. Appareil selon les revendications 1 ou 2 ou 3 ou 4, comportant par ailleurs :
un tampon élastique circulaire (5), dans lequel le tampon élastique est placé autour de la partie extérieure de la bague de gland (1).

9. Appareil selon la revendication 8, dans lequel la bague de gland (1) comporte par ailleurs :
un premier rebord (11) situé sur un premier côté de la bague de gland ; et
un deuxième rebord (12) situé sur un deuxième côté de la bague de gland ; dans lequel les premier et deuxième rebords forment une cavité de forme annulaire (13) sur laquelle le tampon élastique (5) repose.

10. Appareil selon la revendication 9, dans lequel l'anneau à coussinet et le tampon élastique comportent un micro-mécanisme servant à empêcher toute adhésion de la peau sur l'anneau à coussinet et le tampon élastique.

11. Appareil selon la revendication 10, dans lequel :
le micro-mécanisme est sélectionné dans le groupe constitué par des points s'affaissant et bombés, des lignes s'affaissant et bombées croisées, des rainures, des parties saillantes, et une structure irrégulière.

12. Appareil selon la revendication 1, dans lequel :
le dispositif de fixation (3) et la bague de gland (1) comportent une structure de forme elliptique ou de forme obliquement elliptique correspondant à une section en coupe d'un sillon coronal.

13. Appareil selon la revendication 1, dans lequel la bague de gland (1) comporte par ailleurs une entaille (14) à des fins de réception d'un frein de prépuce.

14. Appareil selon la revendication 1 ou la revendication 12, dans lequel :
le dispositif de fixation (3) est en forme de C.

15. Appareil selon la revendication 1 ou la revendication 12, dans lequel :
le dispositif de fixation comporte trois sections connectées ensemble au niveau de deux points d'articulation, les points d'articulation étant décalés l'un par rapport à l'autre de telle sorte qu'ils minimalisent le contact avec un scrotum au cours d'une intervention chirurgicale de circoncision.

16. Appareil selon la revendication 1, dans lequel le moyen de maintien du dispositif de fixation dans une position fermée comporte des blocs de détente en disposition scalariforme supérieur et inférieur (41, 42), chacun des blocs de détente en disposition scalariforme comportant un crochet (13, 14) et un trou, dans lequel les crochets sont dimensionnés et formés à des fins d'adaptation dans les trous, respectivement.

17. Appareil selon la revendication 1, dans lequel :
le moyen de maintien du dispositif de fixation dans une position fermée comporte une vis sur une extrémité du dispositif de fixation et un trou taraudé linéaire sur l'autre extrémité du dispositif de fixation.

18. Appareil selon la revendication 1 ou la revendication 16, comportant par ailleurs un capuchon de protection (6) dimensionné et formé à des fins d'adaptation sur le moyen de maintien du dispositif de fixation dans une position fermée.

19. Appareil selon la revendication 2, dans lequel :
la section en coupe de la rainure (33) est sélectionnée dans le groupe constitué par un arc, un demi-cercle, une semi-ellipse, et un triangle ; et
la section en coupe de la première partie d'accouplement de lame (311A) et de la deuxième partie d'accouplement de lame (311B) combinées est sélectionnée dans le groupe constitué par un arc, un demi-cercle, une semi-ellipse, et un triangle ; et
l'anneau à coussinet (2) comportant une section en coupe correspondant sensiblement à la section en coupe de la rainure (33).

20. Appareil selon la revendication 2, dans lequel :
l'anneau à coussinet (2) est de forme annulaire et la circonférence de celui-ci correspond à celle de la lame (32).

21. Appareil selon les revendications 1, 3 ou 4, dans lequel :
le dispositif de fixation (3) comporte une, deux ou trois lames (32).

22. Appareil selon les revendications 1, 3 ou 4, dans lequel :
des angles arrondis sont mis en oeuvre au niveau des première et deuxième parties d'accouplement de lame (311A, 311B).
